# EUROPEAN PATENT SPECIFICATION

(11) **EP 2 656 837 B1**
(45) Date of publication and mention of the grant of the patent: **15.05.2019**
(21) Application number: 11851122.9
(22) Date of filing: 20.12.2011
(51) Int. Cl.: A61K 9/51, A61K 48/00, A61P 27/02, A61K 31/7105, A61K 31/713, C12N 15/88, C12N 15/87, A61K 9/00, A61K 9/10, A61K 31/7088, A61K 47/00, A61K 38/00

(54) **LIPID NANOPARTICLES FOR TREATING OCULAR DISEASES**
LIPIDNANOPARTIKEL ZUR BEHANDLUNG VON AUGENERKRANKUNGEN
NANOPARTICULES LIPIDIQUES POUR LE TRAITEMENT DE MALADIES OCULAIRES

(30) Priority: 21.12.2010 ES 201031897
(43) Date of publication of application: 30.10.2013
(73) Proprietor: Universidad del Pais Vasco, 48940 Leioa Vizcaya (ES); Universidad Miguel Hernández De Elche, 03202-Elche (Alicante) (ES)
(72) Inventor: RODRÍGUEZ GASCÓN, Alicia, E-48940 Leioa (Vizcaya) (ES); SOLINÍS ASPIAZU, María Angeles, E-48940 Leioa (Vizcaya) (ES); DEL POZO RODRÍGUEZ, Ana, E-48940 Leioa (Vizcaya) (ES); DELGADO SAN VICENTE, Diego, E-48940 Leioa (Vizcaya) (ES); FERNÁNDEZ JOVER, Eduardo, E-03202 Elche (Alicante) (ES)
(74) Representative: ABG Intellectual Property, S.L.
(86) International application number: PCT/ES2011/070883
(87) International publication number: WO 2012/085318

(56) References cited:
- EP-A2- 2 460 516
- WO-A1-00/06120
- WO-A1-02/076441
- WO-A1-2005/120469
- ES-B1- 2 351 756
- RK KHAR ET AL: "Nano-vectors for the ocular delivery of nucleic acid-based therapeutics", INDIAN JOURNAL OF PHARMACEUTICAL SCIENCES, vol. 72, no. 6, 1 November 2010 (2010-11-01), pages 675-88, XP055182875, ISSN: 0250-474X, DOI: 10.4103/0250-474X.84575
- ELIAS FATTAL ET AL: "Nanotechnologies and controlled release systems for the delivery of antisense oligonucleotides and small interfering RNA", BRITISH JOURNAL OF PHARMACOLOGY, vol. 157, no. 2, 2 April 2009 (2009-04-02) , pages 179-194, XP055183700, ISSN: 0007-1188, DOI: 10.1111/j.1476-5381.2009.00148.x
- Robert E Kingston: "CHAPTER 9 Introduction of DNA into Mammalian Cells Contributed by Introduction of DNA into Mammalian Cells", Current Protocols in Molecular Biology, 1 January 2003 (2003-01-01), XP055183720, Retrieved from the Internet: URL:http://webdoc.nyumc.org/nyumc/files/su n-lab/attachments/CPMB.Ch.09.Transfection. pdf [retrieved on 2015-04-16]
- DEL POZO-RODRIGUEZ ET AL.: 'Solid lipid nanoparticles for retinal gene therapy: transfection and intracellular trafficking in RPE cells' INTERNATIONAL JOURNAL OF PHARMACEUTICS vol. 360, no. 1-2, 22 April 2008, pages 177 - 183, XP022853140
- MARUYAMA ET AL.: 'Novel receptor-mediated gene delivery system comprising plasmid/protamine/sugar-containing polyanion ternary complex' BIOMATERIALS vol. 25, no. 16, 21 November 2003, pages 3267 - 3273, XP004490258

## Description

### Field of the Invention

The present invention relates to the use of a lipid nanoparticle system useful for transfecting genetic material in the prevention or treatment of eye diseases.

### Background

In recent years, gene therapy has become an important alternative in the treatment of hereditary or acquired diseases. Although gene therapy was initially proposed as a method for treating hereditary diseases caused by a single gene mutation, i.e., monogenic diseases (cystic fibrosis, hemophilia, Fabry disease, retinopathies, etc.), it is now considered a method which is useful for managing both hereditary and acquired disorders (cancer, AIDS, neurodegenerative diseases, etc.).

Gene therapy consists of introducing genetic material (DNA, RNA or antisense sequences) into target cells for the purpose of modulating the expression of specific proteins which are altered, thus reversing the biological disorder causing the alteration thereof. To develop a product that suitably carries said material, the disease to be treated, the therapeutic gene to be administered and the administration system of that gene must be considered. The system must be capable of protecting the genetic material against enzymatic degradation, facilitating cell uptake and the subsequent release in cell cytoplasm.

One of the most suitable and attractive groups of diseases for the application of gene therapy is the group corresponding to retinal degenerative diseases since there is no effective treatment today for most of them. Besides the harmful characteristics of these diseases such as their hereditary nature, their continuous progression and the lack of treatment thereof, the suitability of the eye as a target for the application of gene therapy must also be pointed out. This organ combines a series of conditions such as its easy accessibility and its exceptional immune environment, distinguishing it as one of the most promising organs for such therapy.

A number of studies to date have approached the use of viral administration systems for treating these diseases with varying degrees of success. Gene administration systems can generally be classified as viral or non-viral vectors. Viral vectors are the most effective, but non-viral vectors are much safer, their production is simpler and more cost effective and they have no limitation in terms of the size of the genetic material to be administered. It is therefore necessary to develop new drugs for gene therapy based on safe non-viral vectors which are capable of improving transfection efficacy.

If the efficacy of non-viral administration systems is successfully increased, these systems can provide a serious alternative to viral vectors since they would solve the problems involved in using viral vectors: risk of toxicity, immunogenicity and mutagenesis derived from an incorrect insertion of the gene administered in the patient genome, limitation of the size of the genetic material to be transfected or difficulties in system preparation processes.

In this sense, nanoparticle-based systems have shown to be useful as transfection systems. In the literature there are various documents describing the use of lipid nanoparticles as vectors for transporting genetic material. Particularly, WO 2005/120469 and the document by Del Pozo-Rodríguez et al. (Int. J. Pharm. 2008, 360, 177-183) refer to lipid nanoparticle systems where the nanoparticles incorporate ionic surfactants for nucleic acid transfection. The document by Del Pozo-Rodriguez et al. (J. Control. Rel., 2009, 133, 52-59) describes lipid nanoparticle systems where the nanoparticles are further coated with a peptide. Nevertheless, the main drawback of most of these systems is low transfection efficacy.

Khar et al. reviewed developments in various nano-vectors for the ocular delivery of nucleic acid-based therapeutics in Indian Journal of Pharmaceutical Sciences 2010, 72(6), 675-688.

EP 2460516, claiming the priority of ES 2351756, refers to the nanoparticles of the present invention and their use for the release of pharmacologically active molecules and for transfecting genetic material into cells and/or tissues.

WO 02/076441 discloses the use of solid nanoparticles obtained from microemulsion precursors for delivering a molecule of interest to the body.

Elias Fattal et al reported the use of non-lipid based systems for controlled released of antisense nucleotides and small interfering RNA in British Journal of Pharmacology 2009, 157(2), 179-194.

The use of diethylaminoethyl (DEAE)-dextran as an alternative technique to other techniques such as electroporation for transfecting DNA into cells is disclosed by Robert E Kingston in Chapter 9.2 of Current Protocols in Molecular Biology 2003.

WO 2005/120469 discloses the use of lipid nanoparticles to deliver nucleic acids and for ocular delivery in gene therapy.

WO 00/06120 discloses the use of a lipid emulsion and solid lipid nanoparticles as gene or drug carrier complexes, and a method to efficiently transfer genes into cells by using said emulsions.

The document by Del Pozo-Rodríguez et al. (International Journal of Pharmaceutics, 2008, 360(1-2), 177-183) describes the preparation of solid lipid nanoparticles (SLN) for retinal gene therapy
In Biomaterials 2003, 25(16), 3267-3273, Maruyama et al. disclose a gene delivery system which comprises a plasmid/protamine/sugar-containing polyanion with enhanced PRT-mediated transfection on hepatic cells.

In view of this data, it is therefore necessary to develop drugs for the treatment of eye diseases by means of gene therapy which are based on safe non-viral vectors with suitable transfection efficacy.

### Brief Description of the Invention

The authors of the present invention have discovered that a lipid nanoparticle system incorporating a polysaccharide in the composition thereof allows achieving an effective level for transfecting genetic material into cells and a high cell viability.

In this sense, the studies conducted by the inventors have demonstrated the capacity of this nanoparticle system for effectively transfecting and allowing the expression of the RS1 gene involved in sex-linked juvenile retinoschisis. Furthermore, *in vivo* assays have clearly shown the capacity of this system to reach retinal cells, which demonstrates the usefulness of this system in the treatment of eye diseases.

This nanoparticle system has also been capable of protecting the genetic material from the action of enzymes, particularly from nucleases, present in biological fluids, preventing its degradation or rupture in this biological medium, thus preventing the genetic material from being released from the nanoparticles before reaching the final target.

Therefore, a first aspect the invention relates to the use of a nanoparticle system, wherein the nanoparticles comprise:
- at least one nucleic acid;
- at least one solid lipid at room temperature;
- at least one cationic surfactant;
- at least one non-ionic surfactant; and
- at least one polysaccharide;
for preparing a medicament for the prevention or
treatment of eye diseases as defined in claim 1.

On the other hand, it has been observed that the incorporation of the polysaccharide together with a positively charged peptide allows synergistically increasing transfection levels when compared with a nanoparticle system that includes only the mentioned peptide or polysaccharide, as well as improving cell viability as described in the examples provided in this application. As a result, an additional aspect of the present invention is the use of the nanoparticles described above which further comprise a positively charged peptide for preparing a medicament for the prevention or treatment of eye diseases as defined in claim 2.

Both the peptide and the rest of the components mentioned above are incorporated within the nanoparticle structure or adsorbed on the surface of said nanoparticles.

In another aspect, the invention relates to a nanoparticle system, where the nanoparticles comprise:
- at least one nucleic acid;
- at least one solid lipid at room temperature;
- at least one cationic surfactant;
- at least one non-ionic surfactant;
- at least one polysaccharide; and
- optionally at least one positively charged peptide;
for use in the prevention or the treatment of eye
diseases as defined in claim 14.

Also is disclosed a method for the prevention or treatment of eye diseases comprising the administration of a nanoparticle system, where the nanoparticles comprise:
- at least one nucleic acid;
- at least one solid lipid at room temperature;
- at least one cationic surfactant;
- at least one non-ionic surfactant;
- at least one polysaccharide; and
- optionally at least one positively charged peptide.

### Description of the Drawings

Figure 1 shows the electrophoresis gel obtained with formulation 2 in which the genetic material condensation capacity of the nanoparticles (1), their protection capacity against DNAses (2) and their release in the presence of SDS (3), are shown.
Figure 2 shows the electrophoresis gel obtained with formulation 3 in which the genetic material condensation capacity of the nanoparticles (1), their protection capacity against DNAses (2) and their release in the presence of SDS (3), are shown.
Figure 3 shows the electrophoresis gel obtained with formulation 5 in which the genetic material condensation capacity of the nanoparticles (1), their protection capacity against DNAses (2) and their release in the presence of SDS (3), are shown.
Figure 4 shows the electrophoresis gel obtained with formulation 6 in which the genetic material condensation capacity of the nanoparticles (1), their protection capacity against DNAses (2) and their release in the presence of SDS (3), are shown.
Figure 5 shows the *in vitro* cell viability and transfection level of formulations 1, 2, 3 and DOTAP liposomal in ARPE-19 cells.
Figure 6 shows a fluorescence microscopy photograph showing the *in vitro* cell viability and transfection level of formulations 1, 2, 3.
Figure 7 shows the *in vitro* cell viability and transfection level of formulations 7-14 in ARPE-19 cells. Figure 8 shows the expression of retinoschisin in ARPE-19 cells detected by immunohistochemistry using formulations 15 (left) and 16 (right).
Figure 9 shows the quantification of retinoschisin in ARPE-19 cells expressed using formulations 15 and 16.
Figure 10 shows a fluorescence microscopy photograph in which the expression of EGFP in rat retina after the intravitreal administration of formulation 3 is shown.
Figure 11 shows a fluorescence microscopy photograph in which the expression of EGFP in rat cornea after the topical administration of formulation 3 is shown.

### Detailed Description of the Invention

The nanoparticle system used in the present invention comprises nanoparticles which have a structure comprising a lipophilic phase and a hydrophilic phase, in which a biologically active molecule can be incorporated. Said nanoparticles are in an aqueous medium although they can optionally be presented as lyophilized or dried products.

In the context of the present invention, the term "nanoparticle" refers to a structure comprising a lipophilic core surrounded by a hydrophilic phase encapsulating the core. The resulting ionic interaction between the different lipophilic and hydrophilic components of the nanoparticle generates independent and observable characteristic physical entities, the mean size of which is equal to or less than 1 µm, i.e., a mean size comprised between 1 and 1000 nm.

"Average size" is understood as the average diameter of the population of nanoparticles comprising the lipophilic phase and the hydrophilic phase. The mean size of these systems can be measured by standard methods that are known by the person skilled in the art and described, for example, in the experimental part below.

The nanoparticles of the system of the invention are characterized by having a mean particle size equal to or less than 1 µm, preferably with a mean size comprised between 1 and 1000 nm, more preferably between 100 and 350 nm. This size allows the nanoparticles to penetrate the cells and administer the biologically active molecule. The mean size of the nanoparticles is mainly influenced by the amount of lipid component (at greater amounts, the resulting size is the same or larger), by the amount of surfactants (at a greater amount or higher molecular weight, the size is the same or smaller), and by parameters of the preparation method, such as the stirring rate speed and type, the temperature of both phases or the duration of the mixing phase.

On the other hand, the nanoparticles can have a surface charge (measured by means of Z potential), the magnitude of which can range from -50 mV to +80 mV.

### Lipid component

The nanoparticle formulation of the present invention comprises at least one solid lipid at room temperature which is part of the nanoparticle core.

In the context of the present invention, "solid lipid at room temperature" is understood as that lipid which remains solid below 45°C, being able to be saturated or unsaturated. Said definition includes triglycerides (for example tristearin), mono- or diglycerides (for example Imwitor®), fatty acids (for example stearic acid), steroids (for example cholesterol) and waxes (for example cetyl palmitate).

In a particular embodiment, the solid lipid at room temperature is selected from acylglycerides, saturated fatty acids with a chain of at least 10 carbon atoms or derivatives thereof and mixtures thereof.

The acylglycerides include monoglycerides, diacylglycerides, triacylglycerides and mixtures thereof. In a preferred embodiment, the acylglycerides are selected from glyceryl palmitostearate (Precirol® ATO5), glyceryl monostearate (Imwitor®900) and glyceryl behenate (Compritol® 888ATO).

In a particular embodiment, the fatty acids are saturated and have a chain of at least 10 carbon atoms. Derivatives of these fatty acids, being understood as those compounds produced as a result of the reaction of the acid group with alcohols or amines, such as for example, the esters or amides of said fatty acids, can also be used. Similarly, those fatty acids, their esters or amides having hydroxyl groups as substituents of the hydrocarbon chain are included in the definition of fatty acid derivatives.

In a preferred embodiment, glyceryl palmitostearate (Precirol® ATO5) is used as a fatty acid derivative.

In another particular embodiment, the nanoparticles further comprise another lipid component, specifically a liquid lipid at a temperature less than 45°C, being able to be saturated or unsaturated. The liquid lipid at room temperature is selected from oils, fatty acids, triglycerides and unsaturated or saturated fatty acid esters having a chain of less than 10 carbon atoms, and the mixtures thereof (for example Miglyol®, soybean oil, isopropyl myristate, castor oil). In a preferred embodiment, Miglyol 212 is used as a liquid lipid.

### Cationic surfactant

The hydrophilic phase of the nanoparticles surrounding the lipophilic core comprises a cationic surfactant. The function of this component is mainly to provide the positively charged nanoparticle that allows its absorption through cationic biological environments or its adsorption thereon.

The term "cationic surfactant" is understood as that compound having a hydrophobic part and a hydrophilic part which forms positively charged ions in a solution and allows obtaining an emulsion.

In a particular embodiment of the invention, the cationic surfactant is selected from linearly or cyclically structured primary, secondary, tertiary and quaternary ammonium salts, and mixtures thereof, such as pyridine or piperazine salts, for example.

Derivatives of these ammonium salts can also be used. Ammonium salt derivatives are understood as those salts incorporating at least two primary, secondary, tertiary and/or quaternary amino groups, such as guanidine, piperazine and imidazole salts for example. This definition would also comprise amino acid salts, such as lysine, arginine, ornithine or tryptophan salts for example. Likewise, this definition would encompass those ammonium salts in which the positive charge is not on the nitrogen atom but rather on a phosphorus atom, such as ditetradecyl(trimethylethylphosphonio)methylphosphonate iodide, ditetradecyl(butyldimethylphosphonio)methylphosphonate iodide, ditetradecyl(dimethylisopropylphosphonio)methylphosphonate iodide) or arsenic(ditetradecyl(trimethylarsonio)methylphosphonate iodide, dioleyl(trimethylphosphonio)methylphosphonate iodide) for example.

In a preferred embodiment of the present invention, the ammonium salts are tetraalkylammonium salts, alkylbenzyl dimethyl ammonium salts or heterocyclic ammonium salts; they are more preferably cetyltrimethylammonium bromide (CTAB), N-[1-(2,3-dioleoyloxy)propyl]-N,N,N-trimethylammonium chloride (DOTAP), or DOTMA (N-[1-(2,3-dioleyloxy)propyl]-N,N,N-trimethylammonium chloride).

### Non-ionic surfactant

The nanoparticles additionally comprise a non-ionic surfactant the main functions of which are to control particle size and confer stability, preventing the rupture of the nanoparticles and the formation of aggregates.

The term "non-ionic surfactant" is understood as that compound having a hydrophobic part and a hydrophilic part which allows obtaining an emulsion.

In a particular embodiment of the present invention, the non-ionic surfactant is selected from polysorbates, polyethylene glycol copolymers and polypropylene glycol copolymers, such as Tween, Span or Poloxamer for example.

### Polysaccharide

The nanoparticle system of the present invention additionally comprises a polysaccharide the main functions of which are to facilitate nanoparticle interaction with the cell surface and to modify the nanoparticle surface charge.

This component can be part of the nanoparticle structure or can be adsorbed on the surface of said nanoparticles. When the polysaccharide is part of the nanoparticle structure, it is in the hydrophilic phase surrounding the lipophilic core together with the cationic surfactant and the non-ionic surfactant.

Alternatively, the polysaccharide can form a complex together with the active ingredient to be incorporated into the nanoparticle formulation by means of ionic interaction. Said complex is contacted with the previously formed nanoparticles, such that the complex formed by the polysaccharide and the active ingredient is adsorbed on the surface of said nanoparticles.

In a particular embodiment, the polysaccharide is selected from chitosans, dextrans, hyaluronic acid, carrageenan, chondroitin, keratan, colominic acid, xanthan, cyclodextrins, salts, derivatives and mixtures thereof. In a preferred embodiment, the polysaccharide is selected from dextran, hyaluronic acid, carrageenan, colominic acid and heparin. According to a particular embodiment, the polysaccharide comprises the binding of at least three monosaccharides and is preferably incorporated within the nanoparticle structure or absorbed on the surface of said nanoparticles.

According to another embodiment, the polysaccharide is not a lipopolysaccharide.

In one embodiment of the present invention, the polysaccharide comprises the binding of at least three monosaccharides, with the proviso that said polysaccharide is not a lipopolysaccharide, and where said polysaccharide is incorporated within the nanoparticle structure or adsorbed on the surface of said nanoparticles.

### Positively charged peptide

The nanoparticle system of the invention can further comprise a positively charged peptide, understanding as such that peptide which ionizes in a solution resulting in a net positive charge.

The authors of the present invention have observed that the combination of the polysaccharide with the mentioned positively charged peptide allows synergistically increasing the transfection levels as well as improving cell viability, as has been clearly shown in the transfection of the pCMS-EGFP plasmid into the human retinal pigment ARPE-19 cell line.

Like the polysaccharide, the positively charged peptide can be part of the nanoparticle structure or can be adsorbed on the surface of said nanoparticles. When the positively charged peptide is part of the nanoparticle structure, it is in the hydrophilic phase surrounding the lipophilic core together with the cationic surfactant, the non-ionic surfactant and the polysaccharide.

Alternatively, the positively charged peptide can form a complex together with the polysaccharide and the nucleic acid to be incorporated in the nanoparticle formulation. Said complex is contacted with the previously formed nanoparticles, such that the complex formed by the polysaccharide, the positively charged peptide and the nucleic acid is adsorbed on the surface of said nanoparticles.

In a particular embodiment of the present invention, the positively charged peptide is selected from nuclear signaling peptides (peptides directed towards the nucleus) and mitochondrial signaling peptides (peptides directed towards the mitochondria), RGD peptides (cell surface recognition peptides containing the arginine-glycine-aspartic acid sequence and variants thereof) and CPP (cell-penetrating peptides). Said peptide is preferably selected from protamines and histones.

In one variant of the invention, the system has a proportion of lipid component which is comprised between 0.1% and 20% by weight with respect to the total weight of the system including water, preferably between 0.5% and 5%. In another variant of the invention, when a liquid lipid at room temperature is incorporated in the nanoparticle formulation, it is in a proportion comprised between 0.1 and 5% by weight with respect to the total weight of the system including water, preferably between 0.5 and 2%.

On the other hand, in another variant of the invention the proportion of cationic surfactant ranges between 0.05% and 5% by weight with respect to the total weight of the system including water, preferably between 0.1% and 2%.

In another variant of the invention, the proportion of non-ionic surfactant is preferably comprised between 0.01 and 10% by weight with respect to the total weight of the system including water, preferably between 0.1 and 3%.

In another variant of the invention, the proportion of polysaccharide is comprised between 0.01 and 10% by weight with respect to the total weight of the system including water, preferably between 0.1 and 3%.

In turn, the proportion of the positively charged peptide when it is present in the formulation is preferably comprised between 0.01% and 10% by weight with respect to the total weight of the system including water, more preferably between 0.1% and 3% by weight.

In all cases the remaining proportion mostly corresponds to purified water where the nanoparticles are dispersed. This does not mean that other ingredients such as viscosity modulators, preservatives, solubilizers, anti-flocculating agents, stabilizers, particularly those which are steric or ionic or surfactant in nature, cannot be present in the nanoparticles, in the aqueous medium where the nanoparticles are dispersed or adsorbed thereon.

### Nucleic acid

The nanoparticles of the present invention provide systems having a high capacity for associating biologically active molecules either within the nanoparticles or adsorbed thereon. Specifically, the nanoparticle system of the invention allows associating up to 100% of the active molecule when it is a plasmid.

The nanoparticles of the present invention include a nucleic acid as a biologically active molecule. In one embodiment of the invention, the nucleic acid can be a single-stranded or double-stranded, natural or synthetic ribonucleotide or deoxyribonucleotide. According to a particular embodiment, the nucleic acid is selected from a DNA plasmid, mRNA, iRNA, microRNA, an oligonucleotide or an antisense sequence. The nucleic acid is preferably a DNA plasmid, such as pEGFP or pRS1 for example.

The nucleic acid can be incorporated within the structure of the nanoparticle or can be adsorbed on the surface of the lipid nanoparticles. According to a particular embodiment, the nucleic acid is adsorbed on the surface of the nanoparticles once they are formed.

The proportion of nucleic acid incorporated in the nanoparticles can be up to 20% by weight with respect to the total weight of the system including water. However, the suitable proportion will depend in each case on the nucleic acid to be incorporated, the indication for which it is used and administration efficiency. In a particular embodiment, the proportion thereof in said system would be between 0.00001% and 20% by weight with respect to the total weight of the system including water.

### Eye diseases

The term "eye diseases" includes any disease, disorder or condition affecting the eye or one of the parts or regions of the eye. The eye generally includes the eyeball and the tissues and fluids making up same, the periocular muscles and the portion of optic nerve which is within or adjacent to the eyeball. The anterior segment of the human eye corresponds to front third of the eye, including the iris, the cornea, the ciliary body and the lens. The posterior segment of the eye includes the vitreous humor, the retina, the choroids and the optic nerve.

The inventors have demonstrated by means of *in vivo* assays that the nanoparticles of the invention are capable of transfecting genetic material (pEGFP) both into retinal cells, particularly at the level of retinal ganglion cells, and into corneal epithelial cells.

Therefore, according to one embodiment the invention relates to the use of the nanoparticle system described above for preparing a medicament for the prevention or treatment of diseases of the posterior segment of the eye, such as retinal degenerative diseases for example.

According to another embodiment, the invention relates to the use of the nanoparticle system described above for preparing a medicament for the prevention or treatment of diseases of the anterior segment of the eye, such as corneal dystrophies for example.

In one embodiment, the invention relates to the use of the nanoparticle system described above for preparing a medicament for the prevention or treatment of eye diseases by means of gene therapy.

The term gene therapy refers to introducing genetic material into a receiving organism for curative purposes. Gene therapy can be used for obtaining the desired therapeutic effect through different strategies depending on the target disease. Gene therapy can be used for providing a gene that is absent in the recipient, or for complementing or substituting the functional defect of a defective gene by introducing a normal copy of the gene into cells. It is also possible to use gene therapy for obtaining the opposite effect, i.e., for inhibiting or blocking the functioning of genes, the intervention of which contributes to diseases development. Alternatively, gene therapy can be used not for substituting or inactivating a gene function, but for introducing the information that allows the cell to synthesize a protein with the desired therapeutic effect.

According to one embodiment, the invention relates to the use of the nanoparticle system described above for preparing a medicament for the prevention or treatment of eye diseases associated with the absence or mutation of a gene encoding one or more proteins.

According to another particular embodiment, the invention relates to the use of the nanoparticle system described above for preparing a medicament for the prevention or treatment of eye diseases associated with the expression or overexpression of a gene encoding one or more proteins.

X-linked juvenile retinoschisis is a hereditary eye disease in which the retina splits spontaneously into two layers and it is one of the most common causes of juvenile macular degeneration in male subjects. The most common clinical manifestation is progressive vision loss during the first two decades of life, worsening again after 50 years of age to the extent that it may cause complete blindness.

This disease is due to the mutations in the RS1 gene of the X chromosome. Said gene encodes the retinoschisin protein which is responsible for maintaining the architecture of the internal retinal layers. Mutations in the RS1 gene in patients with retinoschisis prevent the expression of this protein, the absence of which causes the retinal layers to separate and form small cysts.

The inventors have discovered that the nanoparticles of this invention are capable of transfecting the pCep4-RS1 plasmid into retinal cells (ARPE-19) causing retinoschisin protein synthesis. The use of the nanoparticles of the invention having a polysaccharide and a positively charged peptide provided a retinoschisin concentration which is much higher than that observed for similar systems but lacking the polysaccharide and the peptide, which demonstrates the greater transfection capacity of the nanoparticles used in the invention.

Therefore, in a particular embodiment the invention relates to the use of the nanoparticles described above for preparing a drug for the prevention or treatment of diseases associated with the RS1 gene, preferably X-linked juvenile retinoschisis.

Similarly, these nanoparticles can be used for preventing, improving or treating other eye diseases by means of incorporating the suitable nucleic acid to induce, control or prevent the expression of proteins associated with said disease. By way of illustration, some of the more representative eye diseases for which the nanoparticles of the present invention could be used are shown below. The necessary effect for causing the prevention or the treatment of the indicated disease as well as the nucleic acid which would be incorporated in the nanoparticles of the invention to induce said effect are included in the table.

| Disease | Effect sought | Genetic material to be administered |
|---|---|---|
| Glaucoma | Overexpression of matrix metalloproteinase recombinant protein (MMP1) | Plasmid encoding MMP1 |
| | Overexpression of prostaglandin formation regulator COX-2 | Plasmid encoding COX-2 |
| | Overexpression of neurotrophic factors | Plasmid encoding said neurotrophic factors |
| | Inhibition of the conjunctival growth of fibroblasts | p21 WAF-1/Cip1 |
| Macular degeneration | Inhibition of choroidal neovascularization (inhibition of angiogenic factor expression) | siRNA against angiogenic factors (VEGF, vascular endothelial growth factor) |
| | | Anti-VEGF iRNA |
| | | siRNA against vascular endothelial growth factor receptor-1 (Sirna-27) |
| | | SERPINF1 plasmid encoding the pigment epithelium-derived factor, PEDF |
| | Inhibition of choroidal neovascularization (overexpression of anti-angiogenic factor expression) | Plasmid encoding platelet-derived growth factor (PDGF) |
| | | Ciliary neurotrophic factor (CNTF) |
| | Inhibition of angiogenesis by means of blocking vascular endothelial growth factors | Plasmid encoding sFLT01 and sFLT02 (novel chimeric VEGF-binding molecules) |
| Retinopathies (e.g., diabetic retinopathy, ischemic retinopathy) | Inhibition of retinal neovascularization (inhibition of angiogenic factor expression) | iRNA against angiogenic factors (VEGF) |
| Corneal infections or inflammations (e.g., Keratitis caused by herpes simplex) | Inhibition of corneal neovascularization (inhibition of angiogenic factor expression) | iRNA against angiogenic factors (VEGF) |
| Leber's congenital amaurosis (LCA) and other diseases caused by mutation in RPE65 gene | Expression of the RPE65 gene | Plasmid encoding hRPE65 |
| Corneal surface opacity | Expression of the dnG1 Cyclin gene | dnG1 Cyclin |
| Retinitis pigmentosa (mutation of several genes) | Expression of the gene encoding ciliary neurotrophic factor (CNTF) | CNTF |
| Albinism | Expression of the OA1 gene | OA1 plasmid encoding melasomal GPCR protein |
| Choroideremia | Expression of the CHM gene | CHM plasmid encoding REP-1 cell membrane protein |
| Sex-linked juvenile retinoschisis | Expression of the RS1 gene | RS1 plasmid encoding photoreceptor retinoschisin protein |
| Stargardt's disease | Expression of the ABCA4 gene | ABCA4 plasmid encoding photoreceptor RIN protein |
| Retinoblastoma (retinal cancer) | Expression of tumor-suppressing factor p53 | Plasmid encoding p53 protein |
| | Expression of suicide genes | Suicide gene of herpes simplex virus thymidine kinase/ganciclovir (HSVtk/GCV) |
| Granular corneal dystrophy | Blocking the gene of transforming growth factor beta-induced (TGFBI) protein | siRNA against TGFBI |
| Fuchs' corneal dystrophy | Insertion of the correct copies of the transcription factor 4 (TCF4) gene encoding the E2-2 protein family | Plasmid encoding TCF4 |
| Gelatinous drop-like corneal dystrophy | Expression of the TACSTD-2 (tumor associated calcium signal transducer 2) gene | Plasmid encoding TACSTD-2 |
| Corneal transplant rejection | Inhibition of the cascade of immunological processes leading to transplant rejection. Such treatment can be "*in vivo"* or "ex *vivo*" (genetically modifying the donor's cornea before insertion into the recipient) | Plasmid encoding IL-4 |
| | | Plasmid encoding IL-10 |
| | | Plasmid encoding CTLA4-Ig (cytotoxic T lymphocyte antigen 4-immunoglobulin) |
| Corneal surface opacity associated with mucopolysaccharidosis | Expression of the dnG1 Cyclin gene | dnG1 Cyclin |
| | Expression of the β-glucoronidase gene | Plasmid encoding β-glucoronidase |

Therefore, the invention relates to the use of a nanoparticle system as described above for preparing a medicament for the prevention or treatment of eye diseases selected from: glaucoma; macular degeneration: retinopathy, including diabetic retinopathy and ischemic retinopathy; corneal infections or inflammations, such as herpes simplex; Leber's congenital amaurosis; corneal surface opacity; retinitis pigmentosa; albinism; choroideremia; X-linked juvenile retinoschisis; Stargardt's disease; retinoblastoma; granular corneal dystrophy; Fuchs' corneal dystrophy; gelatinous drop-like corneal dystrophy; corneal transplant rejection; and corneal surface opacity associated with mucopolysaccharidosis.

In the context of the specification, the term "prevention or treatment" means the administration of the nanoparticles according to the invention for maintaining the health in a patient who is suffering or is at risk of suffering eye diseases. Said terms also include the administration of the nanoparticles according to the invention for preventing, improving, relieving or eliminating one or more symptoms associated with eye diseases. In the context of this invention, the term "improving" is understood to mean any improvement in the situation of the treated patient, either a subjective improvement (how the patient feels) or an objective improvement (measured parameters).

The nanoparticles used in the present invention can be part of a pharmaceutical composition. Said pharmaceutical compositions include any solid, semi-solid or liquid (i.e., suspension or dispersion of the nanoparticles of the invention) composition for application by oral, topical, parenteral or ocular route, or any composition in the form of a gel, ointment, cream or balm for administration by topical or ocular route.

According to a particular embodiment, the nanoparticles of the invention or the pharmaceutical composition comprising them is administered by ocular route, preferably by means of subretinal injection, intravitreal injection, subconjunctival injection or by means of topical administration in the eye.

The pharmaceutical compositions can further comprise pH controlling agents, such as, for example, buffer agents preventing the pH of the composition from dropping to values below 5, antioxidant agents inhibiting oxidation of the lipid component, as well as preservatives preventing significant structural changes in the formulation. Depending on their function, these additional components can be present in the phases making up the nanoparticles or in the aqueous medium where they are dispersed or completely or partially adsorbed thereon. The person skilled in the art can determine what additional components can be used and if they are necessary, many of them being commonly used in pharmaceutical and cosmetic compositions.

In a particular embodiment, the nucleic acid is not incorporated within the structure of the nanoparticle but is adsorbed on the surface of the lipid nanoparticles. To that end, said nucleic acid is dissolved in an aqueous solution which is then contacted with the previously obtained lipid nanoparticles.

In another particular embodiment, the polysaccharide and optionally the positively charged peptide when it is present in the formulation are adsorbed on the surface of the nanoparticles together with the nucleic acid. To that end, once the lipid nanoparticles are obtained, a complex comprising the polysaccharide, the nucleic acid and optionally the positively charged peptide is prepared by means of contacting aqueous solutions each of them comprising the three mentioned components with one another. Once said complex is obtained, it is contacted with the previously obtained lipid nanoparticles.

Additionally, other ingredients as defined above, such as viscosity modulators, preservatives, solubilizers, anti-flocculating agents, stabilizers, particularly those which are steric or ionic or surfactant in nature, can be added to nanoparticle system. These components will be added to the lipophilic or hydrophilic phase depending on the nature thereof.

Illustrative examples clearly showing the features and advantages of the invention are described below, nevertheless, they must not be interpreted as limiting the object of the invention as defined in the claims.

### Examples

### Example 1. Preparation of nanoparticles without polysaccharide or peptide by means of the solvent emulsification/evaporation technique (Formulation 1)

A solution of 5% precirol in dichloromethane (2 mL) was prepared. On the other hand, an aqueous solution (10 mL) of 0.4% DOTAP and 0.1% Tween 80 was prepared. The aqueous phase was added to the oily phase, subjecting the mixture to vigorous stirring until obtaining an emulsion. The organic solvent was then evaporated, keeping the emulsion under mechanical stirring for at least 5 minutes, subsequently subjecting it to vacuum for at least 5 minutes. The lipid thus precipitated, a nanoparticle suspension being obtained. After cooling to a temperature between 4 and 8°C, the nanoparticles were filtered by centrifugation and resuspended in purified water.

For preparing the complexes with the genetic material, a solution of 1 µg/µL plasmid (pCMS-EGFP) in distilled water was prepared. The nanoparticle suspension was then contacted with the solution containing the genetic material at a 5:1 ratio (expressed as the weight/weight ratio of DOTAP and DNA) and was kept under stirring for 15 minutes at room temperature.

### Example 2. Preparation of nanoparticles with polysaccharide and without peptide by means of the solvent emulsification/evaporation technique (Formulation 2)

### Lipid nanoparticles:

A solution of 5% precirol in dichloromethane was prepared. On the other hand, an aqueous solution of 0.4% DOTAP and 0.1% Tween 80 was prepared. The aqueous phase was added to the oily phase at a 1:5 ratio, and the mixture was subjected to sonication (Branson Sonifier 250, Danbury) for 30 seconds at 50W. The organic solvent was then evaporated, keeping the emulsion under mechanical stirring for 5 minutes, subsequently subjecting it to vacuum for 15 minutes. After cooling in a refrigerator (4-8°C) for 15 minutes, the obtained nanoparticles were washed by centrifuging 3 times at 3000 rpm for 20 minutes using Amicon® Ultra (Millipore) filters.

### Dextran:pCMS-EGFP complexes:

An aqueous solution was prepared by means of dissolving pCMS-EGFP plasmid (1 µg/µL) in distilled water. On the other hand, another aqueous solution was prepared by means of dissolving dextran having a molecular weight of about 3200 (supplied by Sigma) (1 µg/µL) in distilled water. The two solutions with a dextran:pCMS-EGFP ratio of 5:1 were contacted and kept under stirring for 30 minutes at room temperature.

The nanoparticle suspension was then contacted with the solution containing the complex formed with the genetic material at a 5:1 ratio (expressed as the weight/weight ratio of DOTAP and DNA) and kept under stirring for 15 minutes at room temperature.

### Example 3. Preparation of nanoparticles with polysaccharide and with peptide by means of the solvent emulsification/evaporation technique (Formulation 3)

### Lipid nanoparticles:

A solution of 5% precirol in dichloromethane was prepared. On the other hand, an aqueous solution of 0.4% DOTAP and 0.1% Tween 80 was prepared. The aqueous phase was added to the oily phase at a 1:5 ratio, and the mixture was subjected to sonication (Branson Sonifier 250, Danbury) for 30 seconds at 50W. The organic solvent was then evaporated, keeping the emulsion under mechanical stirring for 5 minutes, subsequently subjecting it to vacuum for 15 minutes. After cooling in a refrigerator (4-8°C) for 15 minutes, the nanoparticles obtained were washed by centrifuging 3 times at 3000 rpm for 20 minutes using Amicon® Ultra (Millipore) filters. Dextran:protamine:pCMS-EGFP complexes:

An aqueous solution was prepared by means of dissolving pCMS-EGFP plasmid (1 µg/µL) in distilled water. On the other hand, another aqueous solution was prepared by means of dissolving dextran having a molecular weight of about 3200 (supplied by Sigma) (1 µg/µL) in distilled water. Additionally, another aqueous solution was prepared by means of dissolving protamine (1 µg/µL) in distilled water. The three solutions were contacted with a dextran:protamine:pCMS-EGFP ratio of 1:2:1 and kept under stirring for 30 minutes at room temperature.

The nanoparticle suspension was then contacted with the solution containing the complex formed with the genetic material at a 5:1 ratio (expressed as the weight/weight ratio of DOTAP and DNA) and kept under stirring for 15 minutes at room temperature.

### Example 4. Preparation of nanoparticles without polysaccharide or peptide by means of the high pressure homogenization technique (Formulation 4)

100 mg of precirol were melted by heating at 70°C. On the other hand, an aqueous solution of 0.4% DOTAP and 0.1% Tween 80 was prepared. A pre-emulsion was prepared by adding the aqueous phase (10 mL) to the melted lipid, subjecting the mixture to vigorous stirring until obtaining an emulsion. The mixture was then subjected to hot high pressure homogenization with a pressure of at least 30 psi and with at least 1 cycle. After cooling in a refrigerator (4-8°C) for 15 minutes, the nanoparticles obtained were washed by centrifuging 3 times at 3000 rpm for 20 minutes using Amicon® Ultra (Millipore) filters.

### Example 5. Preparation of nanoparticles with polysaccharide and without peptide by means of the high pressure homogenization technique (Formulation 5)

### Lipid nanoparticles:

100 mg of precirol were melted by heating at 70°C. On the other hand, an aqueous solution of 0.4% DOTAP and 0.1% Tween 80 was prepared. A pre-emulsion was prepared by adding the aqueous phase (10 mL) to the melted lipid, subjecting the mixture to vigorous stirring until obtaining an emulsion. The mixture was then subjected to hot high pressure homogenization with a pressure of at least 30 psi and with at least 1 cycle. After cooling in a refrigerator (4-8°C) for 15 minutes, the nanoparticles obtained were washed by centrifuging 3 times at 3000 rpm for 20 minutes using Amicon® Ultra (Millipore) filters.

### Dextran:pCMS-EGFP complexes:

An aqueous solution was prepared by means of dissolving pCMS-EGFP plasmid (1 µg/µL) in distilled water. On the other hand, another aqueous solution was prepared by means of dissolving dextran of about 3200 (supplied by Sigma) (1 µg/µL) in distilled water. The two solutions were contacted with a dextran:pCMS-EGFP ratio of 5:1 and kept under stirring for 30 minutes at room temperature.

The nanoparticle suspension was then contacted with the solution containing the complex formed with the genetic material at a 5:1 ratio (expressed as the weight/weight ratio of DOTAP and DNA) and kept under stirring for 15 minutes at room temperature.

### Example 6. Preparation of nanoparticles with polysaccharide and with peptide by means of the high pressure homogenization technique (Formulation 6)

### Lipid nanoparticles:

100 mg of precirol were melted by heating at 70°C. On the other hand, an aqueous solution of 0.4% DOTAP and 0.1% Tween 80 was prepared. A pre-emulsion was prepared by adding the aqueous phase (10 mL) to the melted lipid, subjecting the mixture to vigorous stirring until obtaining an emulsion. The mixture was then subjected to hot high pressure homogenization with a pressure of at least 30 psi and with at least 1 cycle. After cooling in a refrigerator (4-8°C) for 15 minutes, the nanoparticles obtained were washed by centrifuging 3 times at 3000 rpm for 20 minutes using Amicon® Ultra (Millipore) filters.

### Dextran:protamine:pCMS-EGFP complexes

An aqueous solution was prepared by means of dissolving pCMS-EGFP plasmid (1 µg/µL) in distilled water. On the other hand, another aqueous solution was prepared by means of dissolving dextran of about 3200 (supplied by Sigma) (1 µg/µL) in distilled water. Additionally, another aqueous solution was prepared by means of dissolving protamine (1 µg/µL) in distilled water. The three solutions were contacted with a dextran:protamine:pCMS-EGFP ratio of 1:2:1 and kept under stirring for 30 minutes at room temperature.

The nanoparticle suspension was then contacted with the solution containing the complex formed with the genetic material at a 5:1 ratio (expressed as the weight/weight ratio of DOTAP and DNA) and kept under stirring for 15 minutes at room temperature.

### Example 7. Preparation of nanoparticles with polysaccharide and with peptide by means of the solvent emulsification/evaporation technique (Formulation 7)

An aqueous solution was prepared by means of dissolving pCMS-EGFP plasmid (1 µg/µL) in distilled water. On the other hand, another aqueous solution was prepared by means of dissolving dextran having a molecular weight of about 8000 (supplied by Sigma) (1 µg/µL) in distilled water. Additionally, another aqueous solution was prepared by means of dissolving protamine (1 µg/µL) in distilled water. The three solutions were contacted with a dextran:protamine:pCMS-EGFP ratio of 1:3:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 8. Preparation of nanoparticles with polysaccharide and with peptide by means of the solvent emulsification/evaporation technique (Formulation 8)

An aqueous solution was prepared by means of dissolving pCMS-EGFP plasmid (1 µg/µL) in distilled water. On the other hand, another aqueous solution was prepared by means of dissolving dextran having a molecular weight of about 1000 (supplied by Sigma) (1 µg/µL) in distilled water. Additionally, another aqueous solution was prepared by means of dissolving protamine (1 µg/µL) in distilled water. The three solutions were contacted with a dextran:protamine:pCMS-EGFP ratio of 3:1:1 and was kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 9. Preparation of nanoparticles with polysaccharide and with peptide by means of the solvent emulsification/evaporation technique (Formulation 9)

An aqueous solution was prepared by means of dissolving pCMS-EGFP plasmid (1 µg/µL) in distilled water. On the other hand, another aqueous solution was prepared by means of dissolving carrageenan (1 µg/µL) in distilled water. Additionally, another aqueous solution was prepared by means of dissolving protamine (1 µg/µL) in distilled water. The three solutions were contacted with a carrageenan:protamine:pCMS-EGFP ratio of 0.5:2:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 10. Preparation of nanoparticles with polysaccharide and with peptide by means of the solvent emulsification/evaporation technique (Formulation 10)

An aqueous solution was prepared by means of dissolving pCMS-EGFP plasmid (1 µg/µL) in distilled water. On the other hand, another aqueous solution was prepared by means of dissolving colominic acid (1 µg/µL) in distilled water. Additionally, another aqueous solution was prepared by means of dissolving protamine (1 µg/µL) in distilled water. The three solutions were contacted with a colominic acid:protamine:pCMS-EGFP ratio of 0.5:2:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 11. Preparation of nanoparticles with polysaccharide and with peptide by means of the solvent emulsification/evaporation technique (Formulation 11)

An aqueous solution was prepared by means of dissolving pCMS-EGFP plasmid (1 µg/µL) in distilled water. On the other hand, another aqueous solution was prepared by means of dissolving hyaluronic acid (1 µg/µL) in distilled water. Additionally, another aqueous solution was prepared by means of dissolving protamine (1 µg/µL) in distilled water. The three solutions were contacted with a hyaluronic acid:protamine:pCMS-EGFP ratio of 0.1:2:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 12. Preparation of nanoparticles with polysaccharide and with peptide by means of the solvent emulsification/evaporation technique (Formulation 12)

An aqueous solution was prepared by means of dissolving pCMS-EGFP plasmid (1 µg/µL) in distilled water. On the other hand, another aqueous solution was prepared by means of dissolving heparin (1 µg/µL) in distilled water. Additionally, another aqueous solution was prepared by means of dissolving protamine (1 µg/µL) in distilled water. The three solutions were contacted with a heparin:protamine:pCMS-EGFP ratio of 0.1:2:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 13. Preparation of nanoparticles with polysaccharide and without peptide by means of the solvent emulsification/evaporation technique (Formulation 13)

An aqueous solution was prepared by means of dissolving pCMS-EGFP plasmid (1 µg/µL) in distilled water. On the other hand, another aqueous solution was prepared by means of dissolving hyaluronic acid (1 µg/µL) in distilled water. The two solutions were contacted with a hyaluronic acid:pCMS-EGFP ratio of 0.1:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 14. Preparation of nanoparticles with polysaccharide and without peptide by means of the solvent emulsification/evaporation technique (Formulation 14)

An aqueous solution was prepared by means of dissolving pCMS-EGFP plasmid (1 µg/µL) in distilled water. On the other hand, another aqueous solution was prepared by means of dissolving low molecular weight heparin (1 µg/µL) in distilled water. The two solutions were contacted with a low molecular weight heparin:pCMS-EGFP ratio of 0.1:1 and kept under stirring for 30 minutes at room temperature.

The lipid nanoparticle suspension where the lipid nanoparticles are obtained as described in Example 3 was then contacted with the solution containing the complex formed with the genetic material at a 5:1 ratio (expressed as the weight/weight ratio of DOTAP and DNA) and kept under stirring for 15 minutes at room temperature.

### Example 15. Preparation of nanoparticles without polysaccharide or peptide by means of the solvent emulsification/evaporation technique (Formulation 15)

A solution of 5% precirol in dichloromethane (2 mL) was prepared. On the other hand, an aqueous solution (10 mL) of 0.4% DOTAP and 0.1% Tween 80 was prepared. The aqueous phase was added to the oily phase, subjecting the mixture to vigorous stirring until obtaining an emulsion. The organic solvent was then evaporated, keeping the emulsion under mechanical stirring for at least 5 minutes, subsequently subjecting it to vacuum for at least 5 minutes. The lipid thus precipitated, a nanoparticle suspension being obtained. After cooling to temperature between 4 and 8°C, the nanoparticles were filtered by centrifugation and resuspended in purified water.

For preparing the complexes with the genetic material a solution of 1 µg/µL plasmid (pCep4-RS1) in distilled water was prepared. The nanoparticle suspension was then contacted with the solution containing the genetic material at a 6:1 ratio (expressed as the weight/weight ratio of DOTAP and DNA) and kept under stirring for 15 minutes at room temperature.

### Example 16. Preparation of nanoparticles with polysaccharide and with peptide by means of the solvent emulsification/evaporation technique (Formulation 16)

### Dextran:protamine:pCep4-RS1 complex:

An aqueous solution was prepared by means of dissolving pCep4-RS1 plasmid (1 µg/µL) in distilled water. On the other hand, another aqueous solution was prepared by means of dissolving dextran (1 µg/µL) in distilled water. Additionally, another aqueous solution was prepared by means of dissolving protamine (1 µg/µL) in distilled water. The three solutions were contacted with a dextran:protamine:pCep4-RS1 ratio of 1:2:1 and kept under stirring for 30 minutes at room temperature.

The lipid nanoparticle suspension where the lipid nanoparticles are obtained as described in Example 3 was then contacted with the solution containing the complex formed with the genetic material at a 6:1 ratio (expressed as the weight/weight ratio of DOTAP and DNA) and kept under stirring for 15 minutes at room temperature.

### Example 17. Capacity for protecting DNA against DNAse I and induced release with SDS

To know the capacity of the formulations for protecting DNA, the deoxyribonuclease I (DNAse I) enzyme which is capable of hydrolyzing DNA molecules was used. Formulations 1-6 were contacted with DNAse for 30 minutes at 37°C. 1 U of DNAse was used for every 2.5 µg of DNA. After 30 minutes, sodium lauryl sulfate (SDS) was added until a final concentration of 1% to stop the action of the enzyme and release the DNA from the samples. The samples are subsequently analyzed in an agarose gel as defined above in the common methods.

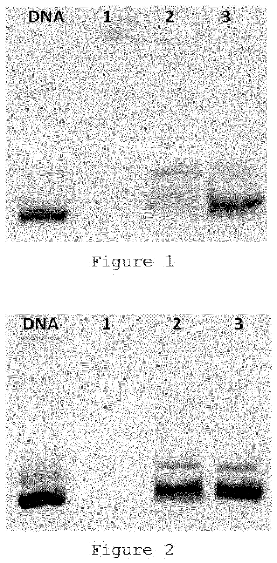

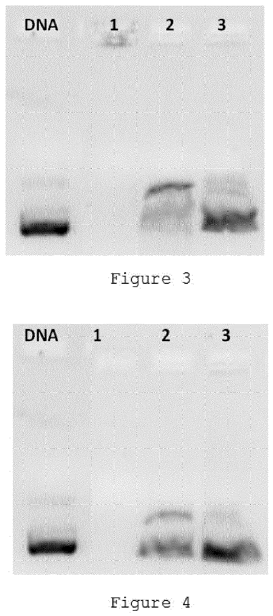

Figures 1 to 4 show the electrophoresis gels showing the DNA condensation capacity, the capacity for protecting against DNAses and the release induced by SDS. It can be confirmed that both the nanoparticles containing the polysaccharide and those containing the polysaccharide-peptide combination are capable of condensing DNA, of protecting it against DNAse and furthermore, the DNA is capable of being released from the complex.

### Example 18. In vitro assays of cell transfection capacity of formulations 1 to 14

The *in vitro* evaluation of formulations 1-14 was carried out in the ARPE-19 cell line (human retinal pigment epithelial cells). These cells were kept in culture in Dulbecco's MEM medium: Ham's Nutrient Mixture F-12, 1:1 Mix (DMEM: F-12) with 10% fetal bovine serum, 0.2% Normocin antibiotic and penicillin. The cell cultures were kept at 37°C in a 5% CO₂ air atmosphere, changing the medium every 2 or 3 days. The transfection studies were conducted in 24-well plates with densities of 30,000 cells per well and were left to incubate until reaching 80-90% confluence. Part of the medium was removed, leaving the necessary volume for covering the entire well, then adding the formulation to be studied. They were left to incubate for 4 hours and more volume of the medium was then added. The amount of vectors added to each well was equivalent to 2.5 µg of DNA.

Cell viability and transfection were evaluated by means of flow cytometry (FACSCalibur™, Becton Dickinson Biosciences, San Jose, USA). EGFP fluorescence was measured at 525 nm (FL1). For each sample, 10,000 events were used. Cell viability was analyzed using the BD Via-Probe™ kit. This kit contains 7-amino-actinomycin (7-AAD) reagent used for non-viable cell exclusion, producing fluorescence in contact with dead cells. 7-AAD fluorescence corresponding to non-viable cells was measured at 650 nm (FL3), 10,000 events being used per sample. Cells subjected to usual culture conditions were used as control cells. Viabilities greater than 80%, a value similar to that obtained with the controls (non-transfected cells) were obtained with all the formulations; this indicates an apparent absence of toxicity. On the other hand, the inclusion of the polysaccharide (formulation 2) and the peptide + polysaccharide combination (formulation 3) increases viability with respect to the formulation that does not include those components (formulation 1). The differences are more notable for formulation 3.

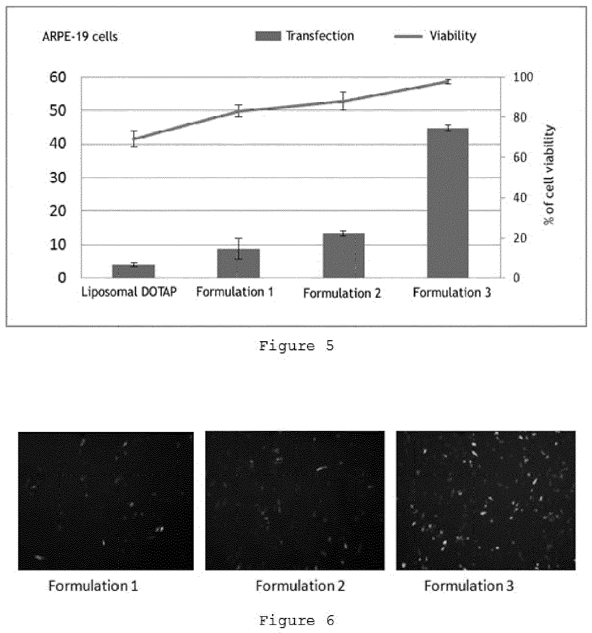

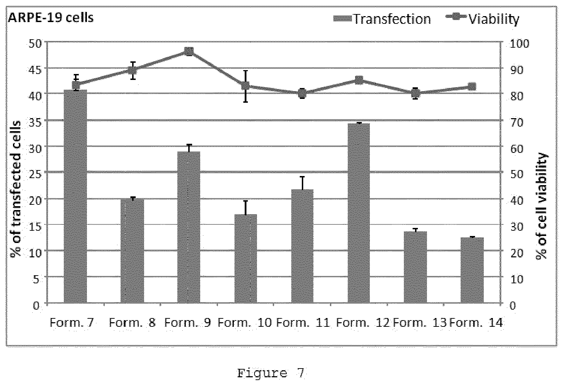

The transfection studies were carried out in ARPE-19 cells. The percentage of transfected cells (% of positive EGFP cells) and cell viability were measured by means of flow cytometry. Figures 5 and 6 show the results corresponding to the cell viability and transfection studies in ARPE-19 cells for formulations 1 to 3, whereas Figure 7 shows the results corresponding to the cell viability and transfection studies in ARPE-19 cells for formulations 7 to 14. A study was conducted with liposomal DOTAP as control.

As can be confirmed, the incorporation of a polysaccharide in the formulation increases transfection with respect to the control group (nanoparticles without polysaccharide or peptide). Nevertheless, the incorporation of a polysaccharide and a peptide in the formulation allows synergistically increasing transfection levels when compared with a nanoparticle system which only includes the mentioned polysaccharide, and improving cell viability.

### Example 19. In vitro assay of the cell transfection capacity of formulations 15 and 16 in the ARPE-19 cell line (human retinal pigment epithelial cells)

The *in vitro* evaluation of formulations 15 and 16 was carried out in the ARPE-19 cell line (human retinal pigment epithelial cells). These cells were kept in culture in Dulbecco's MEM medium: Ham's Nutrient Mixture F-12, 1:1 Mix (DMEM: F-12) with 10% fetal bovine serum, 0.2% Normocin antibiotic and penicillin. The cell cultures were kept at 37°C in a 5% CO₂ air atmosphere, changing the medium every 2 or 3 days. The transfection studies were conducted in 24-well plates with densities of 30,000 cells per well and were left to incubate until reaching 80-90% confluence. Part of the medium was removed, leaving the necessary volume for covering the entire well, then adding the formulation to be studied. They were left to incubate for 4 hours and more volume of the medium was then added. The amount of vectors added to each well was equivalent to 2.5 µg of DNA.

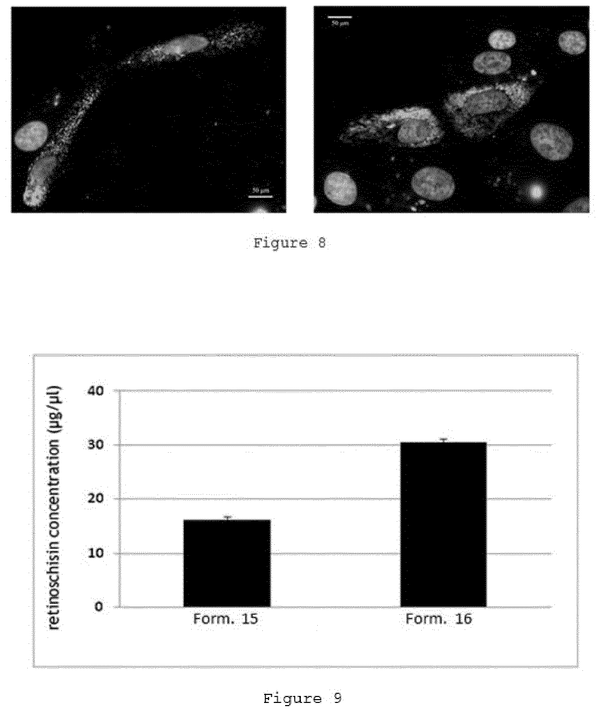

The *in vitro* expression of retinoschisin encoded by the RS1 gene in ARPE-19 cells was detected by means of immunohistochemistry (Figure 8).

The retinoschisin, which was extracted by means of galactose due to the interaction of the discoidin domain of retinoschisin with this saccharide, was further quantified (Figure 9). The extracted protein was quantified with the Bradford technique. As can be confirmed, the incorporation of a polysaccharide and a peptide in the formulation increased transfection with respect to the control group.

### Example 20. In vivo assay of the transfection capacity of the nanoparticles

Adult albino Wistar rats were used to perform the *in vivo* administration of the nanoparticles at the retinal and/or corneal level. Before administration, the animals were intraperitoneally anesthetized with a mixture of ketamine (70 mg/kg, Ketolar®, Pfizer, NY, USA) and xylazine (10 mg/kg, Rompun®, Bayer, Germany). Pupil dilation was then induced in both eyes by applying one drop of 1% tropicamide (1% tropicamide Colicursi®, Alcon-Cusi, Barcelona, Spain), and they were then topically anesthetized with one drop of Double anaesthetic Colircusi® (Alcon-Cusi, Barcelona).

### Administration of lipid nanoparticles in the retina

Both subretinal and intravitreal injections were used for studying the best administration route for the nanoparticles to reach the retina. In both cases, the animals were injected in the left eye with 3 µl of a lipid nanoparticle suspension according to formulations 1 and 3 (0.033 µg pCMS-EGFP µg/µL). To that end, a Hamilton syringe (Hamilton Company, Reno NV, USA) with a needle having an inner diameter of 130 mm (26G caliber) was used. The right eye was used as control. Once the injections were performed, correct retinal vascularization was confirmed by means of direct fundoscopy.

After 96 hours of administration, the animals were euthanized (after being anesthetized with isofluorane). Both eyes were subsequently extracted and fixed in 4% paraformaldehyde in PBS overnight. The retinas were isolated the next day and after washing several times in distilled water, they were mounted on slides (whole mount preparation) with the aid of a fluorescence-specific mounting medium (VectaShield, Vector Laboratories). Finally, the retinas were observed and photographed with a fluorescence microscope (Olympus AX70).

### Topical administration of lipid nanoparticles in the cornea

For the corneal transfection studies, 3 drops of lipid nanoparticle solution were applied in the left eye of albino Wistar rats. The animals were sacrificed after 72 hours, and the eyes were enucleated and fixed in 4% paraformaldehyde overnight. The corneas were isolated the next day and after washing several times in distilled water, they were mounted on slides ("whole mount" preparation) with the aid of a fluorescence-specific mounting medium (VectaShield, Vector Laboratories). Finally, the preparations were observed and photographed with a fluorescence microscope (Olympus AX70).

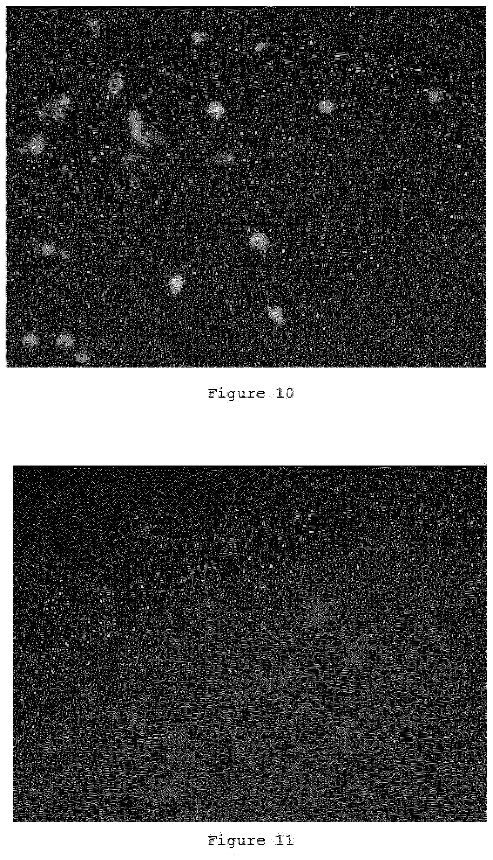

The obtained results show that the administration of lipid nanoparticles with pCMS-EGFP plasmid is capable of inducing effective transfection of pCMS-EGFP into different retinal cell populations, particularly at the level of retinal ganglion cells (Figure 10). In this sense, the intravitreal injection is more efficient for incorporating nanoparticles in retinal cells. On the other hand, the topical administration of lipid nanoparticles with pCMS-EGFP plasmid was also capable of inducing effective transfection of GFP into corneal epithelial cells (Figure 11).

## Claims

1. Use of a nanoparticle system, wherein the nanoparticles comprise:
- at least one nucleic acid;
- at least one solid lipid at room temperature;
- at least one cationic surfactant;
- at least one non-ionic surfactant; and
- at least one polysaccharide;
for preparing a medicament for the prevention or treatment of an eye disease, wherein the eye disease is selected from glaucoma; macular degeneration; retinopathy, including diabetic retinopathy and ischemic retinopathy; corneal infections or inflammations, such as herpes simplex; Leber's congenital amaurosis; corneal surface opacity; retinitis pigmentosa; albinism; choroideremia; X-linked juvenile retinoschisis; Stargardt's disease; retinoblastoma; granular corneal dystrophy; Fuchs' corneal dystrophy; gelatinous drop-like corneal dystrophy; corneal transplant rejection, and corneal surface opacity associated with mucopolysaccharidosis.

2. Use according to claim 1, where the nanoparticles further comprise at least one peptide with a net positive charge.

3. Use according to claim 2, wherein the peptide with a net positive charge is selected from nuclear signaling peptides, mitochondrial signaling peptides, cell surface recognition peptides comprising the arginine-glycine-aspartic acid sequence and cell-penetrating peptides.

4. Use according to any of the preceding claims, wherein the solid lipid at room temperature is selected from monoglycerides, diacylglycerides, triacylglycerides and mixtures thereof.

5. Use according to any of the preceding claims, wherein the cationic surfactant is selected from linearly or cyclically structured primary, secondary, tertiary and quaternary ammonium salts, mixtures thereof and derivatives thereof.

6. Use according to any of the preceding claims, wherein the non-ionic surfactant is selected from polysorbates, polyethylene glycol copolymers, polypropylene glycol copolymers and mixtures thereof.

7. Use according to any of the preceding claims, wherein the polysaccharide is selected from chitosans, dextrans, hyaluronic acid, carrageenan, chondroitin, keratan, colominic acid, xanthan, cyclodextrins, salts, derivatives and mixtures thereof.

8. Use according to any of the preceding claims, wherein the nucleic acid is selected from a DNA plasmid, mRNA, iRNA, microRNA, an oligonucleotide, an antisense sequence and mixtures thereof.

9. Use according to any of the preceding claims, wherein the eye disease is a disease of the posterior segment of the eye.

10. Use according to any of the preceding claims, wherein the eye disease is a disease of the anterior segment of the eye.

11. Use according to any of the preceding claims, wherein the eye disease is due to the absence or mutation of a gene.

12. Use according to any of the preceding claims, wherein the eye disease is due to the expression or overexpression of a gene.

13. Use according to any of the preceding claims, wherein the nanoparticles are administered by means of subretinal injection, intravitreal injection, subconjunctival injection or by means of topical administration in the eye.

14. Nanoparticle system, where the nanoparticles comprise:
- at least one nucleic acid;
- at least one solid lipid at room temperature;
- at least one cationic surfactant;
- at least one non-ionic surfactant;
- at least one polysaccharide; and
- optionally at least one positively charged peptide;
for use in the prevention or the treatment of eye diseases, wherein the eye disease is selected from glaucoma; macular degeneration; retinopathy, including diabetic retinopathy and ischemic retinopathy; corneal infections or inflammations, such as herpes simplex; Leber's congenital amaurosis; corneal surface opacity; retinitis pigmentosa; albinism; choroideremia; X-linked juvenile retinoschisis; Stargardt's disease; retinoblastoma; granular corneal dystrophy; Fuchs' corneal dystrophy; gelatinous drop-like corneal dystrophy; corneal transplant rejection, and corneal surface opacity associated with mucopolysaccharidosis.

## Patentansprüche

1. Verwendung eines Nanopartikelsystems, wobei die Nanopartikel umfassen:
- wenigstens eine Nukleinsäure;
- wenigstens ein bei Raumtemperatur festes Lipid;
- wenigstens ein kationisches Tensid;
- wenigstens ein nichtionisches Tensid; und
- wenigstens ein Polysaccharid;
zum Herstellen eines Medikaments zur Prävention oder Behandlung einer Augenerkrankung, wobei die Augenerkrankung ausgewählt ist aus Glaukom; Makuladegeneration; Retinopathie, einschließlich diabetischer Retinopathie und ischämischer Retinopathie; Infektionen oder Entzündungen der Hornhaut, wie etwa Herpes simplex; Leberscher kongenitaler Amaurose; Trübung der Hornhautoberfläche; Retinitis pigmentosa; Albinismus; Choroideremie; X-chromosomaler juveniler Retinoschisis; Morbus Stargardt; Retinoblastom; granulärer Hornhautdystrophie; Fuchs-Hornhautdystrophie; gelatinöser tropfenförmiger Hornhautdystrophie; Abstoßung von Hornhauttransplantaten und Trübung der Hornhautoberfläche im Rahmen einer Mukopolysaccharidose.

2. Verwendung nach Anspruch 1, wobei die Nanopartikel ferner wenigstens ein Peptid mit einer positiven Nettoladung umfassen.

3. Verwendung nach Anspruch 2, wobei das Peptid mit einer positiven Nettoladung ausgewählt ist aus Kern-Signalpeptiden, mitochondrialen Signalpeptiden, Zelloberflächenerkennungspeptiden, welche die Sequenz Arginine-Glycin-Asparaginsäure umfassen, und zellpenetrierenden Peptiden.

4. Verwendung nach einem der vorangehenden Ansprüche, wobei das bei Raumtemperatur feste Lipid ausgewählt ist aus Monoglyceriden, Diacylglyceriden, Triacylglyceriden und Gemischen derselben.

5. Verwendung nach einem der vorangehenden Ansprüche, wobei das kationische Tensid ausgewählt ist aus linear oder cyclisch strukturierten primären, sekundären, tertiären und quartären Ammoniumsalzen, Gemischen derselben und Derivaten derselben.

6. Verwendung nach einem der vorangehenden Ansprüche, wobei das nichtionische Tensid ausgewählt ist aus Polysorbaten, Polyethylenglykol-Copolymeren, Polypropylenglykol-Copolymeren und Gemischen derselben.

7. Verwendung nach einem der vorangehenden Ansprüche, wobei das Polysaccharid ausgewählt ist aus Chitosanen, Dextranen, Hyaluronsäure, Carrageen, Chondroitin, Keratan, Colominsäure, Xanthan, Cyclodextrinen, Salzen, Derivaten und Gemischen derselben.

8. Verwendung nach einem der vorangehenden Ansprüche, wobei die Nukleinsäure ausgewählt ist aus einem DNA-Plasmid, mRNA, iRNA, miRNA, einem Oligonukleotid, einer Antisensesequenz und Gemischen derselben.

9. Verwendung nach einem der vorangehenden Ansprüche, wobei die Augenerkrankung eine Erkrankung des hinteren Augenabschnitts ist.

10. Verwendung nach einem der vorangehenden Ansprüche, wobei die Augenerkrankung eine Erkrankung des vorderen Augenabschnitts ist.

11. Verwendung nach einem der vorangehenden Ansprüche, wobei die Augenerkrankung durch das Fehlen oder die Mutation eines Gens bedingt ist.

12. Verwendung nach einem der vorangehenden Ansprüche, wobei die Augenerkrankung durch die Expression oder Überexpression eines Gens bedingt ist.

13. Verwendung nach einem der vorangehenden Ansprüche, wobei die Nanopartikel mittels subretinaler Injektion, intravitrealer Injektion, subkonjunktivaler Injektion oder mittels topischer Einbringung in das Auge verabreicht werden.

14. Nanopartikelsystem, wobei die Nanopartikel umfassen:
- wenigstens eine Nukleinsäure;
- wenigstens ein bei Raumtemperatur festes Lipid;
- wenigstens ein kationisches Tensid;
- wenigstens ein nichtionisches Tensid;
- wenigstens ein Polysaccharid; und
- optional wenigstens ein positiv geladenes Peptid;
zur Verwendung bei der Prävention oder der Behandlung von Augenerkrankungen, wobei die Augenerkrankung ausgewählt ist aus Glaukom; Makuladegeneration; Retinopathie, einschließlich diabetischer Retinopathie und ischämischer Retinopathie; Infektionen oder Entzündungen der Hornhaut, wie etwa Herpes simplex; Leberscher kongenitaler Amaurose; Trübung der Hornhautoberfläche; Retinitis pigmentosa; Albinismus; Choroideremie; X-chromosomaler juveniler Retinoschisis; Morbus Stargardt; Retinoblastom; granulärer Hornhautdystrophie; Fuchs-Hornhautdystrophie; gelatinöser tropfenförmiger Hornhautdystrophie; Abstoßung von Hornhauttransplantaten und Trübung der Hornhautoberfläche im Rahmen einer Mukopolysaccharidose.

## Revendications

1. Utilisation d'un système de nanoparticules, dans lequel les nanoparticules comprennent :
- au moins un acide nucléique ;
- au moins un lipide solide à température ambiante ;
- au moins un tensioactif cationique ;
- au moins un tensioactif non ionique ; et
- au moins un polysaccharide ;
pour la préparation d'un médicament pour la prévention ou le traitement d'une maladie oculaire, la maladie oculaire étant l'une parmi : le glaucome ; la dégénérescence maculaire ; la rétinopathie, y compris la rétinopathie diabétique et la rétinopathie ischémique ; les infections ou inflammations cornéennes, telles que l'herpès simplex ; l'amaurose congénitale de Leber ; l'opacité de la surface cornéenne ; la rétinite pigmentaire ; l'albinisme ; la choroïdémie ; la rétinoschisisis juvénile lié au chromosome X ; la maladie de Stargardt ; le rétinoblastome ; la dystrophie cornéenne granulaire ; la dystrophie cornéenne de Fuchs ; la dystrophie cornéenne gélatineuse en goutte ; le rejet des greffes cornéennes, et l'opacité de la surface cornéenne associée avec une mucopolysaccharidose.

2. Utilisation selon la revendication 1, dans laquelle les nanoparticules comprennent en outre au moins un peptide avec une charge positive nette.

3. Utilisation selon la revendication 2, dans laquelle le peptide ayant une charge positive nette est choisi parmi les peptides de signalisation nucléaire, les peptides de signalisation mitochondriaux, les peptides de reconnaissance de surface cellulaire comprenant la séquence d'acide arginine-glycine-aspartique et les peptides à pénétration cellulaire.

4. Utilisation selon l'une quelconque des revendications précédentes, dans laquelle le lipide solide à température ambiante est choisi parmi les monoglycérides, diacylglycérides, triacylglycérides et les mélanges de ceux-ci.

5. Utilisation selon l'une quelconque des revendications précédentes, dans laquelle le tensioactif cationique est choisi parmi les sels d'ammonium primaires, secondaires, tertiaires et quaternaires de structure linéaire ou cyclique, les mélanges de ceux-ci et les dérivés de ceux-ci.

6. Utilisation selon l'une quelconque des revendications précédentes, dans laquelle le tensioactif non ionique est choisi parmi les polysorbates, les copolymères de polyéthylène glycol, les copolymères de polypropylène glycol et les mélanges de ceux-ci.

7. Utilisation selon l'une quelconque des revendications précédentes, dans laquelle le polysaccharide est choisi parmi les chitosanes, les dextranes, l'acide hyaluronique, la carraghénine, la chondroïtine, le kératane, l'acide colominique, le xanthane, les cyclodextrines, les sels, dérivés et mélanges de ceux-ci.

8. Utilisation selon l'une quelconque des revendications précédentes, dans laquelle l'acide nucléique est choisie parmi un plasmide d'ADN, un ARNm, un iARN, un micro ARN, un oligonucléotide, une séquence antisens et les mélanges de ceux-ci.

9. Utilisation selon l'une quelconque des revendications précédentes, dans laquelle la maladie oculaire est une maladie du segment postérieur de l'oeil.

10. Utilisation selon l'une ou l'autre des revendications précédentes, dans laquelle la maladie oculaire est une maladie du segment antérieur de l'oeil.

11. Utilisation selon l'une quelconque des revendications précédentes, dans laquelle la maladie oculaire est due à l'absence ou à la mutation d'un gène.

12. Utilisation selon l'une quelconque des revendications précédentes, dans laquelle la maladie oculaire est due à l'expression ou à la surexpression d'un gène.

13. Utilisation selon l'une quelconque des revendications précédentes, dans laquelle les nanoparticules sont administrées par injection sous-rétinienne, injection intravitréenne, injection sous-conjonctivale ou au moyen d'une administration topique dans l'oeil.

14. Système de nanoparticules, dans lequel les nanoparticules comprennent :
- au moins un acide nucléique ;
- au moins un lipide solide à température ambiante ;
- au moins un tensioactif cationique ;
- au moins un tensioactif non ionique ;
- au moins un polysaccharide ; et
- éventuellement au moins un peptide chargé positivement ;
pour une utilisation dans la prévention ou le traitement de maladies oculaires, la maladie oculaire étant l'une parmi : le glaucome ; la dégénérescence maculaire ; la rétinopathie, y compris la rétinopathie diabétique et la rétinopathie ischémique ; les infections ou inflammations cornéennes, telles que l'herpès simplex ; l'amaurose congénitale de Leber ; l'opacité de la surface cornéenne ; la rétinite pigmentaire ; l'albinisme ; la choroïdémie ; la rétinoschisisis juvénile lié au chromosome X ; la maladie de Stargardt ; le rétinoblastome ; la dystrophie cornéenne granulaire ; la dystrophie cornéenne de Fuchs ; la dystrophie cornéenne gélatineuse en goutte ; le rejet des greffes cornéennes, et l'opacité de la surface cornéenne associée avec une mucopolysaccharidose.
